# EUROPEAN PATENT APPLICATION

(11) **EP 3 162 399 A1**
(43) Date of publication of application: **03.05.2017**
(21) Application number: 15306742.6
(22) Date of filing: 02.11.2015
(51) Int. Cl.: A61M 5/315, A61M 5/31, A61M 5/24

(54) **DRUG DELIVERY DEVICE WITH VARIABLE SIZE DOSE INDEX WINDOW**

(71) Applicant: SANOFI, 75008 Paris (FR)
(72) Inventor: The designation of the inventor has not yet been filed
(74) Representative: Schupp, Bernhard

(57) **Abstract**

The disclosure provides a drug delivery device (1) for selecting and dispensing a number of user variable doses of a medicament having a dose setting mechanism (5), the dose setting mechanism (5) comprises
- a number sleeve (6) adapted to provide a predetermined number of dose markings (6.1) and
- a cover member (7) adapted to cover the number sleeve (6) at least section-wise,
- wherein

the cover member (7) comprises at least a proximal cover member (7.1) and a distal cover member (7.2) axially movable with respect to each other and with respect to the number sleeve (6),
an aperture (7.3) is defined axially between the proximal cover member (7.1) and the distal cover member (7.2) through which one of the dose markings (6.1) is visible, and
the aperture (7.3) is variable in size depending on an axial position of the aperture (7.3) with respect to the number sleeve (6)

## Description

### Technical Field

The disclosure generally relates to a drug delivery device.

### Background

In certain types of drug delivery devices, such as pen-type devices, pre-filled cartridges are used. These cartridges are housed in a cartridge holder or housing. To dispense a certain set dose of a medicament contained in such a cartridge, the drug delivery device has a dose setting element. During drug delivery, a piston rod coupled to the dose setting element presses against a piston (also commonly referred to as a "bung", a "stopper", or a "plunger") contained within the cartridge in order to dispense the medicament through an attached needle assembly.

### Summary

An object of the present disclosure is to provide an improved drug delivery device.

The object is achieved by a drug delivery device according to claim 1.

Exemplary embodiments are provided in the dependent claims.

A drug delivery device for selecting and dispensing a number of user variable doses of a medicament having a dose setting mechanism, wherein the dose setting mechanism comprises
- a number sleeve adapted to provide a predetermined number of dose markings and
- a cover member adapted to cover the number sleeve at least section-wise,
- wherein the cover member comprises at least a proximal cover member and a distal cover member axially movable with respect to each other and with respect to the number sleeve, wherein an aperture is defined axially between the proximal cover member and the distal cover member through which one of the dose markings is visible, and wherein the aperture is variable in size depending on an axial position of the aperture with respect to the number sleeve.

The variable size of the aperture enables an optimally-matched size of the aperture depending on a value of a displayed dose marking. The viewing of a set dose of medicament is thus easier and more user-friendly.

In an exemplary embodiment, the size of the aperture is variable in a proximal direction, wherein the number of dose markings is configured as a dose marking arrangement extending helically about a circumference of the number sleeve such that a value representing the number of dose markings increases in the proximal direction. Thus, the size of the aperture increases with the value of the number of dose markings.

The distal cover member may be configured to extend distally about the circumference of the number sleeve, thereby defining a distal boundary of the aperture. The proximal cover member may be configured to extend proximally about the circumference of the number sleeve, thereby defining a proximal boundary of the aperture. Thus, the size of the aperture is limited by an axial position of the proximal cover member and the distal cover member relative to each other.

In an exemplary embodiment, the dose setting mechanism further comprises a restricting guidance adapted to mechanically restrict an axial movement of the proximal cover member and the distal cover member with respect to the number sleeve respectively. The restricting guidance is further adapted to enable relative axial movement between the distal cover member and the proximal cover member during rotational movement of the number sleeve.

The restricting guidance may comprise a number of corresponding thread sections. For example, a thread section with an increasing thread pitch, in particular increasing in the proximal direction, is arranged on the number sleeve and is adapted to engage a proximal cover thread section that is arranged on the proximal cover member. Furthermore, a thread section with a decreasing thread pitch, in particular decreasing in the proximal direction, is arranged on the number sleeve and is adapted to engage a distal cover thread section that is arranged on the distal cover member. Thus, the thread sections are configured as a multiple thread having different thread pitches. This enables a relative axial movement of the proximal cover member and the distal cover member when the number sleeve is rotated, wherein the proximal cover member engaged to the thread section with the increasing thread pitch moves faster in the proximal direction than the distal cover member engaged to the thread section with the decreasing thread pitch.

Further scope of applicability of the present invention will become apparent from the detailed description given hereinafter. However, it should be understood that the detailed description and specific examples, while indicating exemplary embodiments of the invention, are given by way of illustration only, since various changes and modifications within the spirit and scope of the invention will become apparent to those skilled in the art from this detailed description.

### Brief Description of the Drawings

The present disclosure will become more fully understood from the detailed description given below and the accompanying drawings, which are given by way of illustration only, and do not limit the present disclosure, and wherein:
- Figure 1: is a schematic view of a drug delivery device comprising a dose selecting mechanism and
- Figures 2 to 4: are schematic views of a number sleeve and a cover member in different positions relative to each other.

Corresponding parts are marked with the same reference symbols in all figures.

### Detailed Description

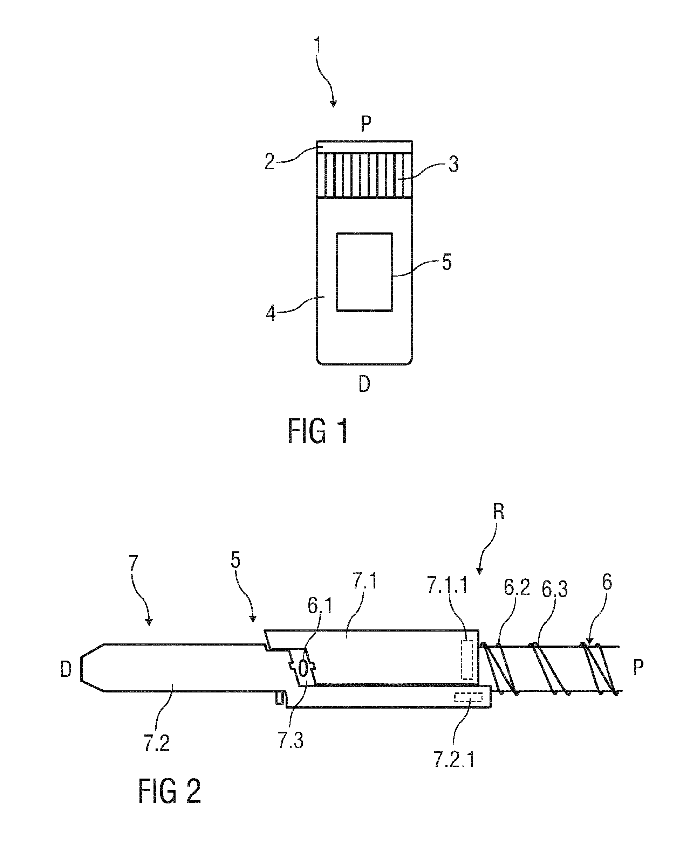

**Figure 1** schematically shows a simplified embodiment of a drug delivery device 1 that is configured as a pen-type device operable to deliver a variable, user-selectable dose of a medicament contained in the drug delivery device 1.

The drug delivery device 1 extends axially between a proximal direction P and a distal direction D. In the present application, the proximal direction P refers to a direction that under use of the drug delivery device 1 is located the furthest away from a drug delivery site of a patient. Correspondingly, the distal direction D refers to a direction that under use of the drug delivery device 1 is located closest to the drug delivery site of the patient.

According to the present embodiment, the drug delivery device 1 comprises a button 2, a dial grip 3, a housing 4 and a dose setting mechanism 5. The drug delivery device 1 may comprise further components that are not shown in the present figures for reasons of clarity.

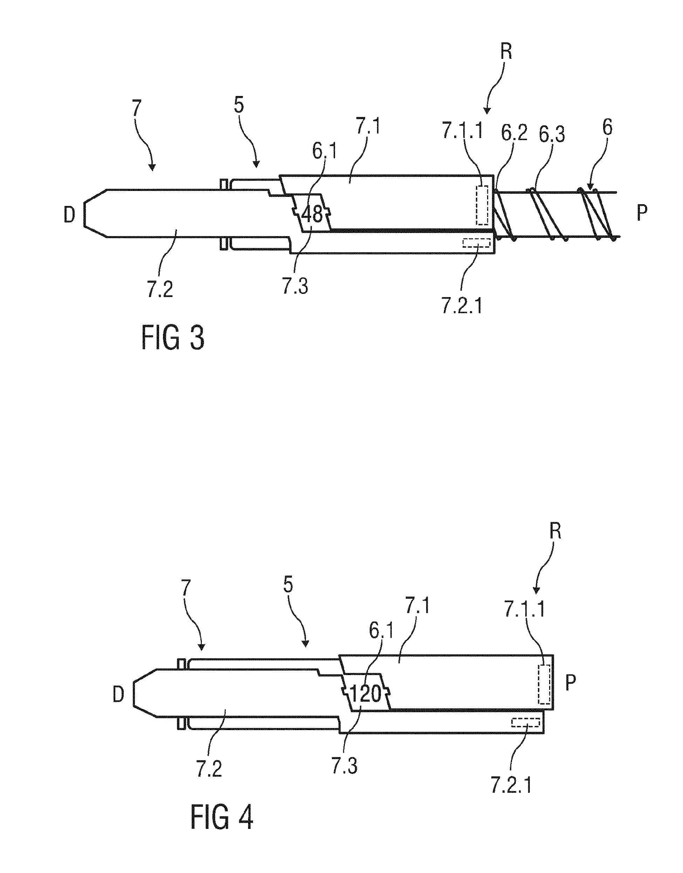

The button 2 is adapted to start a drug delivery process after activating the dose setting mechanism 5. Here, the button 2 forms a proximal end of the drug delivery device 1 and is permanently splined to the dial grip 3 and releasable splined to a number sleeve 6 (illustrated in figures 2 to 5).

The dial grip 3 is adapted to set an individual dose of medicament. Here, the dial grip 3 is a sleeve-like component with a serrated outer skirt and is axially constrained to the housing 4 and rotationally constrained to the button 2.

The housing 4 is a generally tubular element receiving the dose setting mechanism 5.

**Figures 2 to 5** schematically show an exemplary embodiment of the dose setting mechanism 5 comprising the number sleeve 6 and a cover member 7.

According to the illustrated embodiment, the number sleeve 6 is configured as a substantially cylindrical component comprising a number of dose markings 6.1 arranged distally on an outer circumference of the number sleeve 6. In particular, the number of dose markings 6.1 is configured as a dose marking arrangement extending helically about the distal outer circumference of the number sleeve 6, wherein the dose markings 6.1 may be printed directly on the outer circumference of the number sleeve 6 or being engraved there into.

The number sleeve 6 further comprises two thread sections 6.2, 6.3, in particular a thread section 6.2 with an increasing thread pitch and a further thread section 6.3 with a decreasing thread pitch, both arranged proximally behind the number of dose markings 6.1.

According to the illustrated embodiment, the thread sections 6.2, 6.3 together are configured as a multiple outer thread respectively running helically from the distal direction D towards the proximal direction P over the outer circumference of the number sleeve 6. Alternatively, the thread sections 6.2, 6.3 are configured as an inner thread respectively.

The thread sections 6.2, 6.3 differ from each other in their respective thread pitches. The thread section 6.2 with the increasing thread pitch comprises a thread pitch increasing from the distal direction D towards the proximal direction P. However, the further thread section 6.3 comprises a thread pitch decreasing from the distal direction D towards the proximal direction P.

Furthermore, the thread sections 6.2, 6.3 are adapted to engage corresponding thread sections 7.1.1, 7.2.1 arranged on the cover member 7. The thread sections 6.2, 6.3, 7.1.1, 7.2.1 collectively form a restricting guidance R.

According to the present embodiment, the cover member 7 is configured as two-piece component comprising a proximal cover member 7.1 and a distal cover member 7.2. Alternatively, the cover member 7 may comprise more members.

The thread section 6.2 with the increasing thread pitch engages a proximal cover thread section (referred to hereinafter as the "proximal cover thread section 7.1.1") arranged on the proximal cover member 7.1. The further thread section 6.3 with the decreasing thread pitch engages a distal cover thread section (referred to hereinafter as the "distal cover thread section 7.2.1 ") arranged on the distal cover member 7.2. The proximal cover thread section 7.1.1 and the distal cover thread section 7.2.1 may be respectively configured as an inner thread. Alternatively, they are respectively configured as an outer thread, in particular if the thread sections 6.2, 6.3 are configured as an inner thread respectively.

The distal cover member 7.2 extends over a distal portion of the number sleeve 6 covering a distal outer circumference of the number sleeve 6 in the proximal direction P and at least covering the distal outer circumference of the number sleeve 6 section-wise in a circumferential direction. The proximal cover member 7.1 is arranged proximally behind the distal cover member 7.2 and extends over a proximal portion of the number sleeve 6 covering a proximal outer circumference of the number sleeve 6 in the proximal direction P and at least covering the distal outer circumference of the number sleeve 6 section-wise in a circumferential direction.

Both, the distal cover member 7.2 and the proximal cover member 7.1 comprise a recess on their ends facing each other. According to the present embodiment, the recesses are respectively L-shaped and oriented mirror inversed to each other, thereby creating an aperture 7.3 of the cover member 7 through which respectively one of the dose markings 6.1 is visible. The aperture 7.3 is thus limited by a proximal borderline provided by the proximal cover member 7.1 and by a distal borderline provided by the distal cover member 7.2.

Due to the threaded engagement between the distal cover member 7.2, the proximal cover member 7.1 and the number sleeve 6, the aperture 7.3 is variable in size in the proximal direction P or in the distal direction D. This is because the threaded engagement enables a relative axial movement of the distal cover member 7.2 and the proximal cover member 7.1 respectively relative to the number sleeve 6 when the number sleeve 6 is rotated.

Additionally, there may be provided a linear guidance arranged on the housing 4, guiding and restricting the axial movement of the distal cover member 7.2 and the proximal cover member 7.1 with respect to the number sleeve 6. It is conceivable to provide an additional component on the housing 4 and/or on the cover members 7.1, 7.2 to restrict or to prevent a rotational movement of the cover members 7.1, 7.2 with respect to the number sleeve 6.

Because the distal cover member 7.2 is engaged to the further thread section 6.3 with the decreasing thread pitch and the proximal cover member 7.1 is engaged to the thread section 6.2 with the increasing thread pitch, the proximal cover member 7.1 moves faster in the proximal direction P than the distal cover member 7.2 when the number sleeve 6 is rotated in order to set a dose. As a result, the size of the aperture 7.3 increases in the proximal direction P during a proximal movement of the cover member 7 with respect to the number sleeve 6. The aperture 7.3 is smaller in size when displaying a one-digit dose marking 6.1 than when displaying a two- digit or three-digit dose marking 6.1.

The variable size of the aperture 7.3 enables the view of one-digit and more digit dose markings 6.1 with an optimally-matched size. The entire range of dose markings 6.1 can be positioned on the number sleeve 6 with more space saving.

The terms "drug" or "medicament" are used herein to describe one or more pharmaceutically active compounds. As described below, a drug or medicament can include at least one small or large molecule, or combinations thereof, in various types of formulations, for the treatment of one or more diseases. Exemplary pharmaceutically active compounds may include small molecules; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more of these drugs are also contemplated.

The term "drug delivery device" shall encompass any type of device or system configured to dispense a drug into a human or animal body. Without limitation, a drug delivery device may be an injection device (e.g., syringe, pen injector, auto injector, large-volume device, pump, perfusion system, or other device configured for intraocular, subcutaneous, intramuscular, or intravascular delivery), skin patch (e.g., osmotic, chemical, micro-needle), inhaler (e.g., nasal or pulmonary), implantable (e.g., coated stent, capsule), or feeding systems for the gastrointestinal tract. The presently described drugs may be particularly useful with injection devices that include a needle, e.g., a small gauge needle.

The drug or medicament may be contained in a primary package or "drug container" adapted for use with a drug delivery device. The drug container may be, e.g., a cartridge, syringe, reservoir, or other vessel configured to provide a suitable chamber for storage (e.g., short- or long-term storage) of one or more pharmaceutically active compounds. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of a drug formulation (e.g., a drug and a diluent, or two different types of drugs) separately, one in each chamber. In such instances, the two chambers of the dual-chamber cartridge may be configured to allow mixing between the two or more components of the drug or medicament prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drug delivery devices and drugs described herein can be used for the treatment and/or prophylaxis of many different types of disorders. Exemplary disorders include, e.g., diabetes mellitus or complications associated with diabetes mellitus such as diabetic retinopathy, thromboembolism disorders such as deep vein or pulmonary thromboembolism. Further exemplary disorders are acute coronary syndrome (ACS), angina, myocardial infarction, cancer, macular degeneration, inflammation, hay fever, atherosclerosis and/or rheumatoid arthritis.

Exemplary drugs for the treatment and/or prophylaxis of diabetes mellitus or complications associated with diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the term "derivative" refers to any substance which is sufficiently structurally similar to the original substance so as to have substantially similar functionality or activity (e.g., therapeutic effectiveness).

Exemplary insulin analogues are Gly(A21), Arg(B31), Arg(B32) human insulin (insulin glargine); Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Exemplary insulin derivatives are, for example, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin; B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin. Exemplary GLP-1, GLP-1 analogues and GLP-1 receptor agonists are, for example: Lixisenatide / AVE0010 / ZP10 / Lyxumia, Exenatide / Exendin-4 / Byetta / Bydureon / ITCA 650 / AC-2993 (a 39 amino acid peptide which is produced by the salivary glands of the Gila monster), Liraglutide / Victoza, Semaglutide, Taspoglutide, Syncria / Albiglutide, Dulaglutide, rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An exemplary oligonucleotide is, for example: mipomersen / Kynamro, a cholesterol-reducing antisense therapeutic for the treatment of familial hypercholesterolemia.

Exemplary DPP4 inhibitors are Vildagliptin, Sitagliptin, Denagliptin, Saxagliptin, Berberine.

Exemplary hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Exemplary polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 / Synvisc, a sodium hyaluronate.

The term "antibody", as used herein, refers to an immunoglobulin molecule or an antigen-binding portion thereof. Examples of antigen-binding portions of immunoglobulin molecules include F(ab) and F(ab')₂ fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region.

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, and multispecific antibodies (e.g., diabodies, triabodies, tetrabodies), minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Exemplary antibodies are anti PCSK-9 mAb (e.g., Alirocumab), anti IL-6 mAb (e.g., Sarilumab), and anti IL-4 mAb (e.g., Dupilumab).

The compounds described herein may be used in pharmaceutical formulations comprising (a) the compound(s) or pharmaceutically acceptable salts thereof, and (b) a pharmaceutically acceptable carrier. The compounds may also be used in pharmaceutical formulations that include one or more other active pharmaceutical ingredients or in pharmaceutical formulations in which the present compound or a pharmaceutically acceptable salt thereof is the only active ingredient. Accordingly, the pharmaceutical formulations of the present disclosure encompass any formulation made by admixing a compound described herein and a pharmaceutically acceptable carrier.

Pharmaceutically acceptable salts of any drug described herein are also contemplated for use in drug delivery devices. Pharmaceutically acceptable salts are for example acid addition salts and basic salts. Acid addition salts are e.g. HCl or HBr salts. Basic salts are e.g. salts having a cation selected from an alkali or alkaline earth metal, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are known to those of skill in the arts.

Pharmaceutically acceptable solvates are for example hydrates or alkanolates such as methanolates or ethanolates.

Those of skill in the art will understand that modifications (additions and/or removals) of various components of the substances, formulations, apparatuses, methods, systems and embodiments described herein may be made without departing from the full scope and spirit of the present invention, which encompass such modifications and any and all equivalents thereof.

### List of References

- 1: drug delivery device
- 2: button
- 3: dial grip
- 4: housing
- 5: dose setting mechanism
- 6: number sleeve
- 6.1: dose marking
- 6.2: thread section
- 6.3: further thread section
- 7: cover member
- 7.1: proximal cover member
- 7.1.1: proximal cover thread section
- 7.2: distal cover member
- 7.2.1: distal cover thread section
- 7.3: aperture

- D: distal direction
- P: proximal direction
- R: restricting guidance

## Claims

1. A drug delivery device (1) for selecting and dispensing a number of user variable doses of a medicament having a dose setting mechanism (5), wherein the dose setting mechanism (5) comprises
- a number sleeve (6) adapted to provide a predetermined number of dose markings (6.1) and
- a cover member (7) adapted to cover the number sleeve (6) at least section-wise,
- wherein
the cover member (7) comprises at least a proximal cover member (7.1) and a distal cover member (7.2) axially movable with respect to each other and with respect to the number sleeve (6),
wherein an aperture (7.3) is defined axially between the proximal cover member (7.1) and the distal cover member (7.2) through which one of the dose markings (6.1) is visible, and
the aperture (7.3) is variable in size depending on an axial position of the aperture (7.3) with respect to the number sleeve (6).

2. The drug delivery device (1) according to claim 1, wherein the size of the aperture (7.3) is variable in size in a proximal direction (P).

3. The drug delivery device (1) according to claim 2, wherein the number of dose markings (6.1) is configured as a dose marking arrangement extending helically about a circumference of the number sleeve (6)such that the value represented by the number of dose markings (6.1) increases in the proximal direction (P).

4. The drug delivery device (1) according to claim 2 or 3, wherein the distal cover member (7.2) is configured to extend distally about the circumference of the number sleeve (6), thereby defining a distal boundary of the aperture (7.3).

5. The drug delivery device (1) according to any one of the claims 2 to 4, wherein the proximal cover member (7.1) is configured to extend proximally about the circumference of the number sleeve (6), thereby defining a proximal boundary of the aperture (7.3).

6. The drug delivery device (1) according to any one of the preceding claims, wherein the dose setting mechanism (5) further comprises a restricting guidance (R) adapted to
- mechanically restrict an axial movement of the proximal cover member (7.1) and the distal cover member (7.2) with respect to the number sleeve (6) respectively and
- enable relative axial movement between the distal cover member (7.2) and the proximal cover member (7.1) during rotational movement of the number sleeve (6).

7. The drug delivery device (1) according to claim 6, wherein the restricting guidance (R) comprises a number of corresponding thread sections (6.2, 6.3, 7.1.1, 7.2.1).

8. The drug delivery device (1) according to claim7, wherein a thread section (6.2) with an increasing thread pitch is arranged on the number sleeve (6) and adapted to engage a proximal cover thread section (7.1.1) arranged on the proximal cover member (7.1).

9. The drug delivery device (1) according to claim 7 or 8, wherein a thread section (6.3) with a decreasing thread pitch is arranged on the number sleeve (6) and is adapted to engage a distal cover thread section (7.2.1) arranged on the distal cover member (7.2).
